# EUROPEAN PATENT APPLICATION

(11) **EP 1 147 770 A1**
(43) Date of publication of application: **24.10.2001**
(21) Application number: 01118965.1
(22) Date of filing: 05.06.1997
(51) Int. Cl.: A61K 31/42, A61K 38/15, A23K 1/17

(54) **Virginiamycin mixture**

(30) Priority: 01.07.1996 US 20512 P
(62) Divisional of application: 97922002.7
(71) Applicant: PFIZER INC., New York, N.Y. 10017 (US)
(72) Inventor: Carson, James William Pat.Dept.Pfizer Global Res., Groton, Connecticut 06340 (US); Chapin, Frederic William Pat.Dept.Pfizer Global, Groton, Connecticut 06340 (US); Fahrenholz, Charles Hollett Pat.Dept.Pfizer, Groton, Connecticut 06340 (US)
(74) Representative: Hayles, James Richard

(57) **Abstract**

A mixture comprising: a) virginiamycin; b) a pharmaceutically acceptable and substantially anhydrous wetting agent including sodium lauryl sulfate; c) a sufficient amount of pharmaceutically acceptable buffering agent to provide a pH of from about 3.0 to about 7.0 when said mixture is added to water; and d) from about 0.5 weight percent to about 10.0 weight percent colloidal silicon dioxide, wherein the ratio of the weight percent of (b) to the weight percent of (a) percent by weight is at least about 1.5:1. The mixture is designed to be added to water to produce a stable suspension of the virginiamycin, which can then be applied to, for example, feed grain.

## Description

### Background of the Invention

The present invention relates to a mixture comprising virginiamycin.

Virginiamycin antibiotics, in their most effective form, include both M and S components. Coccito, Micro. Rev., 43, 145 (1979). Virginiamycin has been used as an antibacterial and in the prevention of lactic, acidosis (e.g. United States Patents 5,137,900 and 5,242,938 and The Merck Index, 12th Edition, pages 1707-1708). It has also been used in the form of a feed additive to improve growth in poultry, swine, and cattle. A possible mechanism for its use as a growth promotant could relate to an inhibition of intestinal flora. Coccito, supra. Virginiamycin's widely accepted use stems from having low toxicity, minimal production of resistant mutant strains, quick degradation in feces, and minimal tissue retention.

Wettable powders have been used for the administration of various insecticides and herbicides.

### Summary of the Invention

The present invention relates to a mixture comprising:
a) virginiamycin;
b) a pharmaceutically acceptable surfactant including sodium lauryl sulfate;
c) a sufficient amount of pharmaceutically acceptable buffering agent to provide a pH of from about 3.0 to about 7.0 when said mixture is added to water; and
d) from about 0.5 weight percent to about 10.0 weight percent colloidal silicon dioxide,

wherein the ratio of the weight percent of (b) to the weight percent of (a) is at least about 1.5:1.

### Detailed Description

The mixture comprising virginiamycin is in the form of a wettable-type powder. The powder mixture can be added to water, including some forms of hard water, to produce a stable suspension of the virginiamycin, which can then be, for example, delivered directly to a patient (e.g orally) or applied to, for example, food stuffs such as feed grain. The feed grain is then fed to animals, for example livestock and poultry, thereby administering the virginiamycin. The mixture should preferably be substantially anhydrous so as to maximize the shelf life of the mixture prior to forming the water suspension.

Virginiamycin can be produced using known methods, for example, fermentation. For example, virginiamycin M can be produced by fermentation of a streptomyces species originally isolated from Indian soil samples and deposited with the American Type Culture Collection, 12301 Parklawn Drive, Rockville, Maryland 20852, United States of America on July 29, 1986 and assigned ATCC number 53527, United States Patent 5,242,938.

Dose levels of virginiamycin are known in the art and can vary due to a number of factors, including, for example, compound activity, the route of administration, the time of administration, disease severity, excretion rate, combination of other drugs, as well as the age. body weight, sex, diet, and general health of the patient being treated. Generally, for example, with a human subject, the daily effective dose can range from about 1.0 mg to about 1500 mg, preferably about 10 mg to about 500 mg, in single or divided doses. For a domestic animal, the effective dose can range from about 5 to about 200 parts per million per volume of food. United States Patent 5,242,938.

For veterinary purposes, dosage levels known in the art can also be found, for example, in the 1996 Feed Additive Compendium, Miller Publishing Co. (1996), pp. 324-328.

The claimed mixture comprises virginiamycin, preferably substantially pure, and a pharmaceutically acceptable surfactant, sodium lauryl sulfate (SLS), preferably substantially anhydrous and granulated. The ratio of the weight percent of SLS to the weight percent of virginiamycin should be at least about 1.5 to 1, preferably about 1.54 to 1. Substantially pure virginiamycin has an activity of about 200% (plus or minus 20%) as measured by methods known in the art (Gossele, et al., Analyst, 116, 1373 (1991) and Blain, et al., Analyst, 119, 361 (1994).

The powder mixture also includes a pharmaceutically acceptable anti-caking agent, colloidal silicon dioxide, which can act as scavenger for water as well as a wetting and suspending agent for the virginiamycin. The amount of colloidal silicon dioxide can range from 0.5 weight percent of the mixture to about 10.0 weight percent of the mixture, preferably about 2.0 weight percent.

A pharmaceutically acceptable buffer is also included, for example phosphate or citrate buffers, preferably citric acid/sodium citrate. The amount of buffer should be sufficient to provide a pH of from about 3.0 to about 7.0, preferably from about 4.0 to about 5.5 when the mixture is added to water. Maintenance of pH permits the virginiamycin to be suspended in a water environment with minimal effect on its activity.

The powder mixture is added to water to form a stable suspension of the virginiamycin, which is then applied to, for example, feed grain. The amount of mixture dispensed in the water is approximately 4.5% by weight of the suspension.

A pharmaceutically acceptable water soluble filler material can also be included. Examples include sugars, such as lactose and dextrose, preferably lactose. Such fillers may also effect the flowability of the mixture.

A pharmaceutically acceptable anti-foaming agent, which is, for example, an emulsion and will not substantially effect the pH of the resulting suspension, can also be included in the anhydrous mixture, for example, polydimethylsiloxane. Once the suspension is made, the anti-foaming agent minimizes the amount of foam caused by the SLS upon agitation of the suspension. This can be important if, for example, the suspension is being sprayed and a pump is needed to drive the suspension through the spraying apparatus. The amount of anti-foaming agent in the mixture can range from about 0.5 weight percent to about 10.0 weight percent, preferably about 2.8 weight percent.

The mixture should preferably be maintained as substantially anhydrous prior to forming the suspension in order to minimize the breakdown of the components of the mixture. As a result of minimizing breakdown, the shelf life of the mixture can be maximized.

The mixture is formed by adding its ingredients together and thoroughly blending them together. Once the mixture is formed, a suspension can be made by adding the mixture to water. The resulting suspension is substantially stable with minimal sedimentation of virginiamycin falling out of the suspension for about a day with periodic stirring or with little or no stirring of the suspension for about a couple hours, preferably about six hours once the suspension has been properly formed.

A pharmaceutically acceptable indicator dye can also be included, depending upon acceptable food and drug regulations. The dye, for example, FD&C blue No. 1 powder, would give a blue color to the suspension and, in tum, to feed grain sprayed therewith. Thus, the treated grain can be readily identified.

A preferred formulation of the claimed invention is the following:

| **INGREDIENT** | **FORMULA** | **% W/W** |
|---|---|---|
| Virginiamycin Feed Grade* (activity = about 200%) | not available | 18.8 |
| Sodium Lauryl Sulfate | C₁₂H₂₅NaO₄S | 30.0 |
| Colloidal Silicon Dioxide | SiO₂ | 2.0 |
| Citric Acid, anhydrous, fine granular | C₆H₈O₇ | 20.0 |
| Sodium Citrate anhydrous powder | C₆H₅N₂₃O₇ | 20.0 |
| Antifoam AF emulsion | [-(CH₃)₂SiO₂]ₙ | 2.8 |
| FD&C Blue No. 1 powder | FDA Lot # AG4275 Wamer-Jenkinson Co. Code # 05601 | 0.4 |
| Lactose* anhydrous, 80 mesh | C₁₂H₂₂O₁₁ | 6.0 |

| | | |
|---|---|---|
| * Virginiamycin and lactose calculated to provide 40% virginiamycin activity. Amounts will vary depending on purity of virginiamycin. | | |

The following are intended as non-limiting examples of the present invention.

### Example 1

The below listed ingredients were combined and mixed together until a uniform mixture was formed.

| **INGREDIENT** | **% w/v** | **AMOUNT** |
|---|---|---|
| Virginiamycin Feed Grade 213% | 0.94 | 9.40g |
| Sodium Lauryl Sulfate | 1.50 | 15.00g |
| Colloidal Silicon Dioxide | 0.10 | 1.00g |
| Citric Acid U.S.P. | 1.00 | 10.00g |
| Sodium Citrate U.S.P. | 1.00 | 10.00g |
| Antifoam A.F. | 0.10 | 1.00g |

A suspension was formed by adding the above mixture to 900 ml of distilled water in a 1 liter graduated cylinder until dispersion was complete. Then additional water was added to bring the total volume to 1 liter, pH 4.24.

### Example 2

The below listed ingredients were combined and mixed together until a uniform mixture was formed.

| **INGREDIENT** | **% w/v** | **AMOUNT** |
|---|---|---|
| Virginiamycin Feed Grade 213% | 0.94 | 9.40g |
| Sodium Lauryl Sulfate | 1.50 | 15.00g |
| Colloidal Silicon Dioxide | 0.10 | 1.00g |
| Citric Acid U.S.P. | 1.00 | 10.00g |
| Sodium Citrate U.S.P. | 1.00 | 10.00g |
| Antifoam A.F. | 0.10 | 1.00g |

A suspension was formed by adding the above mixture to 900 ml of distilled water in a 1 liter graduate until dispersion was complete. Then additional water was added to bring the total volume to 1 liter. pH 4.30.

### Example 3

The below listed ingredients were placed in a mixer and mixed for 15 minutes to form a base mixture.

| **INGREDIENT** | **% w/v** | **AMOUNT** |
|---|---|---|
| Sodium Lauryl Sulfate | 36.9 | 4500.0 g |
| Citric Acid | 24.6 | 3000.0 g |
| Sodium Citrate | 24.6 | 3000.0 g |
| Anhydrous Lactose, 80 Mesh (Sheffield) | 7.4 | 900.0 g |
| Antifoam A. F. Emulsion | 3.4 | 420.0 g |
| Colloidal Silicon Dioxide | 2.6 | 300.0 g |
| FD&C Blue No. 1 Powder | 0.5 | 60.0 g |

A. 1126.8 g of virginiamycin animal feed grade (213% activity) (18.8%) was added to 4873.2 g of base mixture (81.2%) in a mixer and mixed for 15 minutes.
B. 956.1 g of virginiamycin animal feed grade (205% activity) (19.5%) was added to 3943.2 g of base mixture (80.5%) in a mixer and mixed for 15 minutes.

## Claims

1. A mixture comprising:
a) virginiamycin;
b) a pharmaceutically acceptable surfactant including sodium lauryl sulfate;
c) a sufficient amount of pharmaceutically acceptable buffering agent to provide a pH of from about 3.0 to about 7.0 when said mixture is added to water; and
d) from about 0.5 weight percent to about 10.0 weight percent colloidal silicon dioxide,
wherein the ratio of the weight percent of (b) to the weight percent of (a) percent by weight is at least about 1.5:1.

2. The mixture of claim 1, further including a pharmaceutically acceptable anti-foaming agent.

3. The mixture of claim 1, further including a pharmaceutically acceptable dye.

4. The mixture of claim 1, wherein said mixture is substantially anhydrous.

5. Feed grain treated with a water suspension of a mixture comprising:
a) virginiamycin;
b) a pharmaceutically acceptable surfactant including sodium lauryl sulfate;
c) a sufficient amount of pharmaceutically acceptable buffering agent to maintain said suspension at a pH of from about 3.0 to about 7.0;
d) from about 0.5 weight percent to about 10.0 weight percent colloidal silicon dioxide,
wherein the ratio of the weight percent of (b) to the weight percent of (a) percent by weight is at least about 1.5:1.

6. The feed grain of claim 5, said mixture further including a pharmaceutically acceptable dye.

7. The feed grain of claim 5, further including a pharmaceutically acceptable anti-foaming agent in said mixture.

8. A method of treating feed grain with virginiamycin, comprising
(1) forming a mixture including:
a) virginiamycin,
b) a pharmaceutically acceptable surfactant including sodium lauryl sulfate,
c) a sufficient amount of pharmaceutically acceptable buffering agent to provide a pH of from about 3.0 to about 7.0 when said mixture is added to water, and
d) from about 0.5 weight percent to about 10.0 weight percent colloidal silicon dioxide, wherein the ratio of the weight percent of (b) to the weight percent of (a) percent by weight is at least about 1.5:1;
(2) suspending said mixture in water to form a suspension; and
(3) applying said suspension to feed grain.

9. The method of claim 8, further including a pharmaceutically acceptable anti-foaming agent in said mixture.

10. The method of claim 8, further including a pharmaceutically acceptable dye in said mixture.

11. The method of claim 8, further including drying the feed grain after the applying step.
